# EUROPEAN PATENT APPLICATION

(11) **EP 0 781 557 A1**
(43) Date of publication of application: **02.07.1997**
(21) Application number: 95830515.3
(22) Date of filing: 13.12.1995
(51) Int. Cl.: A61K 31/715

(54) **Use of suleparoide in the prevention of endothelitis and other endothelial reactions induced by therapeutic intravenous infusions**

(71) Applicant: Società Italo-Britannica L. Manetti-H. Roberts & C. S.p.A., 50127 Firenze (IT)
(72) Inventor: Bononi, Loris J., I-54020 Massa Carrara (IT); Spignoli, Giacomo, I-50122 Firenze (IT)
(74) Representative: Bardini, Marco Luigi

(57) **Abstract**

Use of a glycosaminoglycan known as "suleparoide" for the preparation of a medicament for the prevention of endothelitis, endothelial damages and subendothelial ssclerotic reactions caused by intravenous infusions of therapeutic substances such as antitumoral drugs, parenteral nutrition, etc..

## Description

The present invention refers to a glycosaminoglycan known as "suleparoide" (HHS.5), prepared in a pharmaceutical form for parenteral use, endowed with protective activity towards endothelitis, endothelial damages and subendothelial sclerotic reactions, caused by intravenous infusions with therapeutic purposes (e.g. cancer chemotherapy, parenteral nutrition, etc.).

Cancer is the most important cause of death in the industrialized countries after the cardiovascular diseases. Its prevalence and incidence are continuously growing because of the increase in life expectation determined by the reduced mortality due to infective and cardiovascular diseases.

In the last years, interesting results have been achieved in cancer chemotherapy, which can dispose of powerfull drugs with high selectivity of action towards tumoral cells. The anticancer chemotherapy allows to achieve good results in the treatment of certain cancers, for which high percentages of success can be obtained, such as cancer of lymphocytic system, urogenital tract, gastrointestinal apparatus, mammary glands, etc..

However, the use of cytostatic drugs is hampered by their poor tolerability because of the side effects on rapidly growing normal tissues, such as gastrointestinal mucosa, hematopoietic system, germinal cells, etc..

Another problem which frequently limits the use of cytostatic drugs, is the damage of the endothelial cells of the superficial vein (usually, of the elbow) through which the drug has been infused. The damage can reveal itself as a simple endothelial irritation (with skin erythema and spontaneous or induced pain along the vessel) or as a clinically evident phlebitis (with perivasal phlogosis, eventually complicated by a thrombophlebitis).

The chemical damage of the venous endothelial cells is always complicated by a perivascular fibrotic reaction, even in absence of phlebitic symptomatology. The clinical result is the disappearance of the vein, which can be hardly found for a new venous infusion. This obliges the physician or the nurse to look for another vein in other less comfortable regions (hand, leg, foot, etc.) and increases the (already heavy) sufferings of the patient. Moreover, in many cases, the impossibility to find other venous accesses obliges the physician to install a central venous catheter, which cause further sufferings to the patients and brings the risk of severe septicaemia.

It is therefore of great scientific interest the surprising invention that the heparinoid "suleparoide" (HHS-5) is able to protect the vascular endothelium toward the chemical damage induced by anticancer drugs.

In addition, suleparoide has been found therapeutically effective in preventing the infusion flebitis provoked by parenteral nutrition.

The invention can be considered surprising, because suleparoide is known for its antithrombotic and profibrinolytic activity, and its ability to protect the endothelium against chemical damage by anticancer chemotherapeutics could not be foreseen because it is based on biochemical and physiopathogenetic mechanism which are completely different from those responsible for the antithrombotic activity.

The discovery has a relevant therapeutic interest, because suleparoide is the first compund pharmacologically active and therapeutically effective in the prevention of endothelitis and subendothelial sclerotic reactions provoked by venous infusion. At present, there is no convalidated pharmacological therapy for this problem and the auxiliary aids are usually palliative, such as dilution of the drugs, washout of the vessel with saline after the infusion, use of a central venous catheter, etc.. It is noteworthy to mention that these aids do not guarantee an effective protection or cause other side effects (e.g. septicaemia).

Recently, a clinical trial has shown that heparin can reduce the incidence of phlebitis induced by cancer chemotherapy (Thurlimann B., Bachmann I., *Effective prevention of chemotherapy-induced phlebitis by low-dose heparin: a prospective randomised trial*. Ann. Oncol. 1992;3:311-3)

However, heparin can be handled with difficulty in patients with cancer, because of its side effects on long-term treatment (haemorrhagies on the site of infusion, osteoporosis, hypersensibility, alopecia, thrombocytopenia, etc.)

Suleparoide is a glycosaminoglycan, chemically described as polyhexuronyl-1-4-α-D-N-acetyl-(N-sulphonyl-) glucosamine 6-O-sulfate, sodium salt. It is devoid of the heparin-like anticoagulant, but it is endowed with *in vivo* antithrombotic and profibrinolytic activity.

The hexuronyl residues are represented by: D-glucoronic, L-iduronic, L-iduronyl-2-sulfate.

Suleparoide (HHS-5), which can be generally defined as an heparan sulfate, according to the definition proposed by Lindhal and Lane (heparin-like structure with a content of N-acetyl higher than 20%), is a white powder, slightly hygroscopic. The chemical structure is: 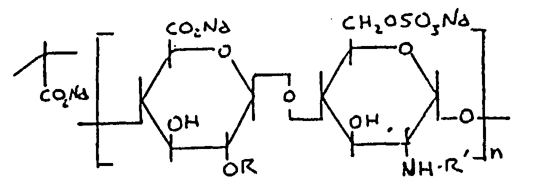 where
- R: = H o -SO₃Na
- R': = -SO₃Na o CH₃-CO-(about 50% each)
- n (average): = about 20
The molecular structure is:
- for R' = CH₃CO,R = H:: (C₁₄H₁₉NO₁₄SNa₂)n, MW = 503 D
- for R' = CH₃CO,R = SO₃Na:: (C₁₄H₁₈NO₁₇SNa₃)n, MW = 605 D
- The mean molecular weight is:: MW = 10.250 + 1.350 D (MW = weighed mean molecular weight).

Suleparoide is chemically obtained as a N-acetylated derivative of heparin, through a reaction of a N-desulfated heparin (heparamine) with acetic anidride in alkaline environment. The final product has the following chemical characteristics:

| | |
|---|---|
| Anticoagulant activity | < 10 IU/mg |
| Content of N-acetyl (as CH₃CO-) (corresponding to a degree of N-acetylation between 40 and 60%) | 3-4,7% W/W |
| Content of O-acetyl | absent |
| Uronic acid (after hydrolysis) | 31 ± 1% |
| Hexosamines (after hydrolysis) | 32 ± 1% |
| Organic sulfate (as S) | 8 ± 1% |
| Molar rate uronic/hexosamines/S/acetyl | 1:1:1.5-1.7:0.4-0.6 |
| Specific optic rotation | > + 35° |

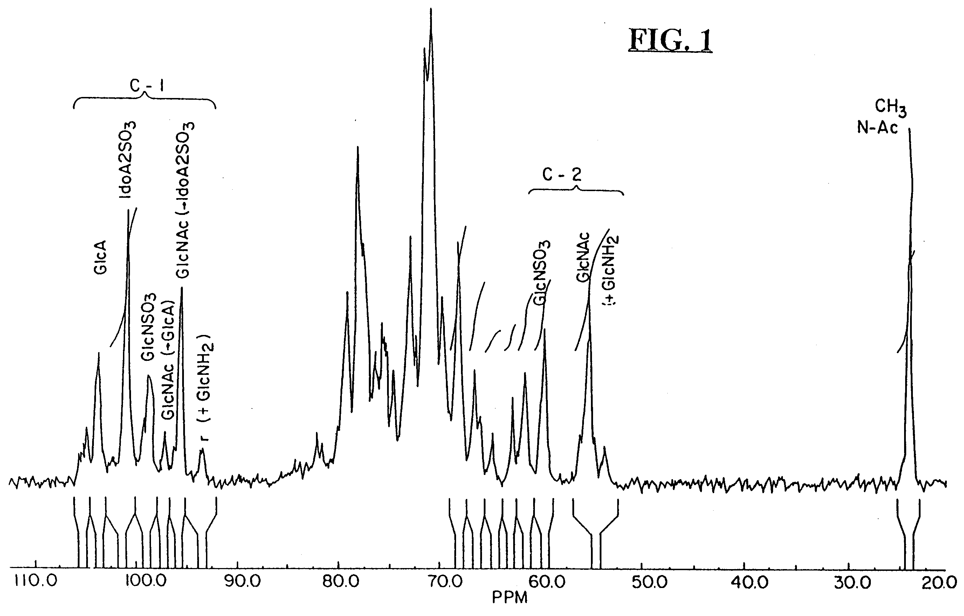

Figure 1 shows the N.M.R. (Nuclear Magnetic Resonance) spectra of suleparoide (HHS-5), with the identification of the relative chemical groups. In the ¹³C-NMR spectra obtained in D₂O, operating at 75 Mhz, the peak relative to the N-acetyl is clearly evident (main difference vs heparin), as well as the peaks of glucosamine N-sulfate (GlcNSO₃) and N-acetylglucosamine (GlcNac) in the zone identified as C-2, and the peaks of glucoronate (GlcA) and iduronyl-C⁵-sulfate (IdoA₂SO₃) in the zone C-1.

Suleparoide (HHS-5) and its method of production are disclosed in EP-A-0413248.

Suleparoide is a glycosaminoglycan, generically called as "heparan sulfate", according to the statement of Gallagher et al (1985) that: "...the term "heparin" should be restricted to a polysaccharide in which more than 80 percent of the glucosamine residues are N-sulfated and the concentration of O-sulfate groups exceeds that of the N-sulfate groups. It was recommended that all other related polysaccharides should be referred to as heparan sulfate" (*Biochemical Journal 230, 665-674, 1985)* Suleparoide exerts antithombotic activity, as shown in different experimental models of arterial and venous thrombosis. This activity is supported by a dose-dependent activation of fibrinolysis, that has been demonstrated both in animals and in patients with cardiovascular diseases (chronic obstructive arteriopathy, chronic cerebrovascular disease, atheroschlerotic coronaropathy, etc.). The profibrinolytic activity of suleparoide is supported by an increase in the plasma levels of the tissue-type plasminogen activator (tPA) and a reduction in its inhibitor (PAI). Because of its profibrinolytic activity, suleparoide is indicated in the diseases caused by impaired fibrinolysis and, particularly, in the treatment and prevention of thrombotic complications of cardiovascular diseases.

Since Suleparoide has been studied and developed for its antithrombotic activity and effects on coagulation and fibrinolysis, it was surprising to discover that suleparoide is endowed with other than the antithrombotic activity, e.g. the protective effects towards the endothelitis and endothelial damage provoked by venous infusion of anticancer chemotherapeutics and by parenteral nutrition.

### Effects of Suleparoide on the endothelitis and flebosclerosis induced by anticancer chemotherapy.

The Suleparoide's protective effects on the venous endothelium has been experimentally studied in a model oon endothelitis induced by intravenous administration of anticancer drugs.

Male Wistar rats (180-220 gr.b.w.) have been treated with the anticancer drugs that more frequently induce in patients phlogistic reactions of the endothelium and reactive perivasal sclerosis. The endothelial damage has been measured on the basis of the number of detached endothelial cells (EC), found in plasma 2 hours after the treatment with the anticancer drugs. The protective activity of suleparoide has been evaluated, giving the coumpound 30 minutes before the cytostatic drugs. The plasma has been obtained by centrifugation at 2,100 rpm x 10 min from blood collected with siliconized material and added with trisodic citrate 3.8% 1:10. The precipitate obtained after removal of platelets with ADP and centrifugation at 3,000 rpm x 15 min., was resuspended in 3 ml of a 0.5 procaine solution, and the Ecs counted with a Burker chamber. The number of EC has been evaluated as a mean of 4 different determinations and expressed as n° CE/9 µl.

Groups of 18-20 rats have been treated with the following anticancer drugs: vinorelbine (0.3 mg/kg), vindesine (0.04 mg/kg), dacarbazine (2 mg/kg), carmustine (BCNU) (2 mg/kg), doxorubicin (1 mg/kg), cyclophosphamide (0.1 mg/kg).

All the drugs provoked an endothelial damage as shown by a statistically significant increase in plasma ECs; these data confirm the irritant effects of anticancer chemotherapeutics. The administration of suleparoide dose-dependently reduced the number of ECs at doses ranging between 0.01 and 10 mg/kg: the activity of the glycosaminoglycan towards the different drugs is reported in Table 1. In the same Table, the effects of other glycosaminoglycans are reported; it is noteworthy to underline that no other compound was active (dermatan sulfate, condroitin sulfate, hyaluronic acid).

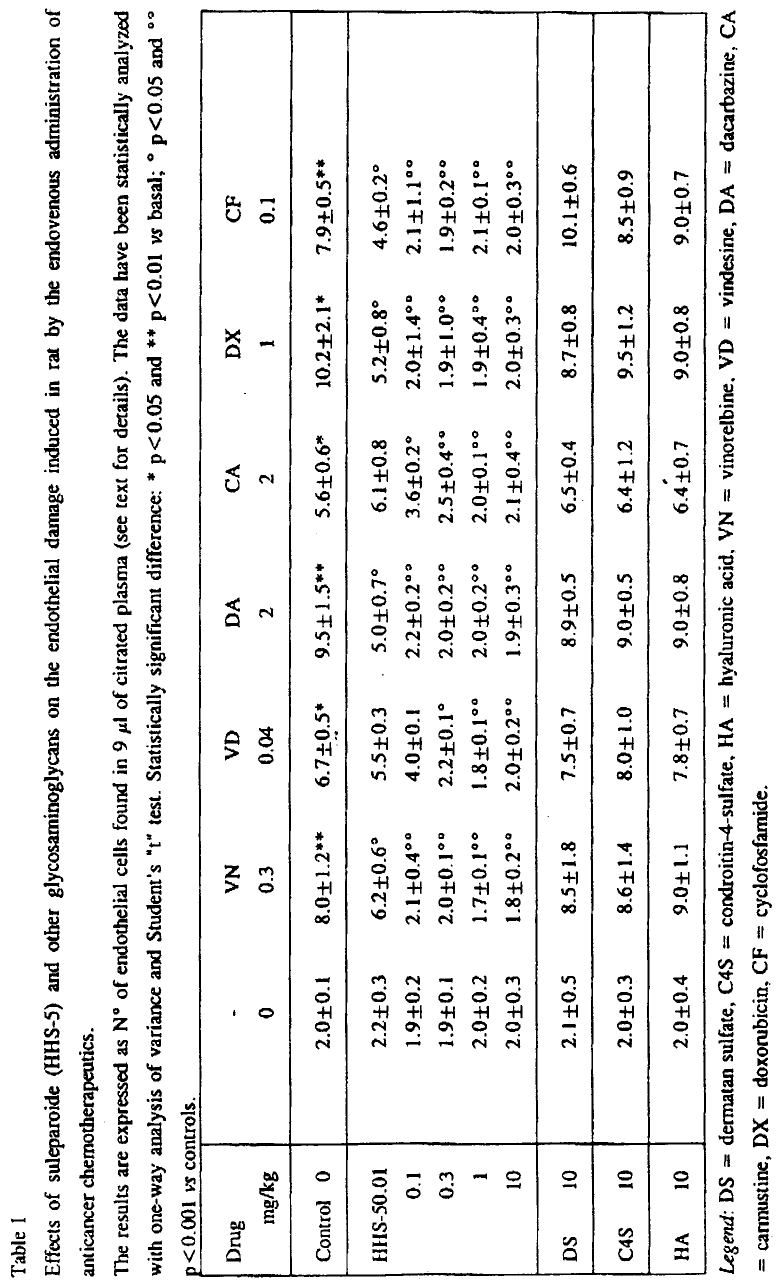

The therapeutic efficacy of suleparoide has been confirmed in a double-blind placebo-controlled randomized multicenter clinical trial, which enrolled 182 patients receiving multiple-day chemotherapy for cancer. The cytostatics most often used were vinorelbine, vindesine, dacarbazine, mechloretamine, antrhacyclines, bleomycin. Patients were randomly assigned to Group (A) or Group (B), and treated with 2 vials of placebo or suleparoide, respectively. One vial was administered before starting of chemotherapy infusion and another one at the end of infusion. The treatment was repeated before any other infusion. The therapeutic efficacy of suleparoide has been evaluated with the following parameters:
a) **Phlebitis**: the total number of phlebitis observed in each group;
b) **Phleb./pat (%)**: the incidence of phlebitis for 100 patients (or, the probability of each patient to develop at least one phlebitis during a therapy lasting on average 4 infusions);
c) **Phleb./Infus. (%)**: the incidence of phlebitis for 100 infusions (or the probability of each infusion to be complicated by a phlebitis);
d) **Vein lost**: the total number in each group of the veins lost for perivascular reactive fibrosis;
e) **Vein lost/pat (%)**: the incidence of vein lost for 100 patients (or, the probability of each patient to loss at least one vein during a therapy lasting on average 4 infusions);
f) **Vein lost/infus.(%)**: the incidence of vein lost for 100 infusions (or the probability of each infusion to be complicated by the loss of one vein).

The results of the clinical trial are summarized in Table 2, and they show that:
- 52.69% of patients given with anticancer chemotherapy and placebo develop a phlebitis;
- 87.10% of patients loss one vein at least during the therapy lasting 4 infusions.

However, when the infusions of chemotherapeutic agents are preceded by suleparoide intravenous administration:
- only 4.49% of patients develop a phlebitis;
- only 23.60% of patients loss a vein during the therapy.

**Table 2**

| Number of infusions in patients under treatment with cancer chemotherapy and treated with placebo or suleparoide i.v. and incidence of phlebitis and veins lost. | | |
|---|---|---|
| | *Placebo* | *Suleparoide* |
| N° patients | 93 | 89 |
| Infusions | 380 | 346 |
| Inf./pat. | 4.09 ± 0.130 | 3.89 ± 0.183 |
| Phlebitis | 49 | 4** |
| Phleb./pat. (%) | 52.69 | 4.49** |
| Phleb./Infus. (%) | 12.89 | 1.16** |
| Vein lost | 81 | 21* |
| Vein lost/pat. (%) | 87.10 | 23.60** |
| Vein lost/infus. (%) | 21.32 | 6.07* |

| | | |
|---|---|---|
| The parametric data were statistically analyzed with the one-way analysis of variance and Student's "t" test. The non-parametric data were analyzed with chi2-two-tailed test corrected by Yates and the one-way Kruskall-Wallis test. Statistically significant difference: * p<0.05 and | | |
| **p<0.01 vs placebo. | | |

### Effects of Suleparoide in the endothelitis and phlebosclerosis induced by parenteral nutrition therapy

Parenteral nutrition provides life-sustaining therapy for patients who cannot take adequate nutrition by mouth and who consequently are at risk for the debilitating complications of malnutrition. Partial and short-term parenteral nutrition can be provided via a peripheral vein, but the repeated use of a venous access can be complicated by an infusion phlebitis. In another double-blind clinical multicenter trial, 146 patients who needed parenteral nutrition (massive small bowel resection, moderate to severe acute pancreatitis, necrotizing enterocolitis, GI major surgery, extensive burns, etc.) were ruled into the study. Patients have been randomly assigned to Group (A) or Group (B), and treated with 2 vials of placebo or suleparoide, respectively. One vial was administered before starting of parenteral infusion, and another one immediately after. The treatment was repeated before any other infusion. The therapeutic efficacy of suleparoide has been evaluated with the same above-mentioned parameters:
a) **Phlebitis**: the total number of phelibitis observed in each group;
b) **Phleb./pat (%)**: the incidence of phlebitis for 100 patients (or, the probability of each patient to develop at least one phlebitis during a therapy lasting on average 8 infusions);
c) **Phleb./Infus.(%)**: the incidence of phlebitis for 100 infusions (or the probability of each infusion to be complicated by a phlebitis);
d) **Vein lost**: the total number of veins lost in each group;
e) **Vein lost/pat.(%)**: the incidence of vein lost for 100 patients (or, the probability of each patient to loss at least one vein during a therapy lasting on average 8 infusions);
f) **Vein lost/infus.(%)**: the incidence of vein lost for 100 infusions (or the probability or each infusion to be complicated by the loss of one vein).

The results of the clinical trial are summarized in Table 3, and they show that:
- 47.22% of patients given with parenteral nutrition and placebo develop a phlebitis;
- 70.83% of patients loss one vein at least during the therapy lasting on average 8 infusions.

However, when the infusion of the nutritive preparation was preceded by suleparoide intravenous administration:
- only 4.05% of patients develop a phlebitis;
- only 16.22% of patients loss a vein during the therapy.

**Table 3**

| Incidence of phlebitis and veins lost in patients under parenteral nutrition via a peripheral vein, treated with placebo or suleparoide i.v. before each infunsion. | | |
|---|---|---|
| | *Placebo* | *Suleparoide* |
| N° patients | 72 | 74 |
| Infusions | 618 | 623 |
| Inf./pat. | 8.58 ± 0.756 | 8.42 ± 0.671 |
| Phlebitis | 34 | 3* |
| Phleb./pat. (%) | 47.22 | 4.05* |
| Phleb./Infus. (%) | 5.50 | 0.48* |
| Vein lost | 51 | 12* |
| Vein lost/pat. (%) | 70.83 | 16.22** |
| Vein lost/infus. (%) | 8.25 | 1.93* |

| | | |
|---|---|---|
| The parametric data were statistically analyzed with the one-way analysis of variance and Student's "t" test. The non-parametric data were analyzed with chi2-two-tailed test corrected by Yates and the one-way Kruskall-Wallis test. Statistically significant difference: * p<0.05 and | | |
| **p<0.01 vs placebo. | | |

A typical pharmaceutical preparation of suleparoide is in the form of an ampoule containing 100 mg of this drug in a sterile physiological solution. The preparation is used to prevent endothelitis and other endothelial reactions induced by therapeutic intravenous infusion and is administered intravenously at a dose of 50-200 mg before and after the therapeutic infusion.

## Claims

1. Use of suleparoide for preparing a medicament for preventing endothelitis and other endothelial reactions induced by therapeutic intravenous infusions.

2. Use according to claim 1, wherein said therapeutic intravenous infusion is a chemotherapic drug infusion.

3. Use according to claim 1, wherein said therapeutic intravenous infusion is a parenteral nutrition infusion.

4. Use according to claim 1, wherein suleparoide is provided in the form of ampoule containing 100 mg of suleparoide in a sterile physiological solution for intravenous administration.

5. Use according to claim 1, wherein suleparoide is administered at a dose of 50-200 mg intravenously before and after said therapeutic infusion.
